(19) Europäisches Patentamt
European Patent Office
Office européen des brevets

(11) Veröffentlichungsnummer: **0 241 004 B1**

(12) **EUROPÄISCHE PATENTSCHRIFT**

(45) Veröffentlichungstag der Patentschrift: **30.12.92**

(51) Int. Cl.[5]: **C07K 5/06**, C07K 5/08, C12Q 1/34

(21) Anmeldenummer: **87105182.7**

(22) Anmeldetag: **08.04.87**

# (54) Chromophore Peptide, Verfahren zu ihrer Herstellung und ihre Verwendung zum Nachweis der peptidylglycin-alpha-amidierenden Monooxygenase.

(30) Priorität: **11.04.86 DE 3612302**

(43) Veröffentlichungstag der Anmeldung:
**14.10.87 Patentblatt 87/42**

(45) Bekanntmachung des Hinweises auf die Patenterteilung:
**30.12.92 Patentblatt 92/53**

(84) Benannte Vertragsstaaten:
**AT BE CH DE ES FR GB GR IT LI LU NL SE**

(56) Entgegenhaltungen:

**CHEMICAL ABSTRACTS, Band 76, 1972, Zusammenfassung Nr. 109632c, Seite 186, Columbus, Ohio, US; G.P. SACHDEV et al.: "Fluorescence studies on the interaction of pepsin with its substrates", & BIOCHEMISTRY 1972, 11(6), 1080-6**

**CHEMICAL ABSTRACTS, Band 93, 1980, Zusammenfassung Nr. 64476n, Seite 368, Columbus, Ohio, US; G.S. PENNY et al.: "Interaction of dansylated petidyl chloromethanes with trypsin, chymotrypsin, elastase and thrombin", & BIOCHEMISTRY 1980, 19(13), 2888-94**

**CHEMICAL ABSTRACTS, Band 106, 1987, Zusammenfassung Nr. 210288h, Seite 326, Columbus, Ohio, US; O.S. RESHETOVA et al.: "Analysis of N-dansyl peptide methyl esters by means high performance liquid chromatography and mass spectrometry", & BIOORG. KHIM. 1987, 13(3), 320-37**

(73) Patentinhaber: **HOECHST AKTIENGESELLSCHAFT**
**Postfach 80 03 20**
**W-6230 Frankfurt am Main 80(DE)**

(72) Erfinder: **König, Wolfgang, Dr.**
**Eppsteiner Strasse 25**
**W-6238 Hofheim am Taunus(DE)**
Erfinder: **Müllner, Hubert, Dr.**
**Berliner Ring 20**
**W-6233 Kelkheim (Taunus)(DE)**
Erfinder: **Glauder, Jan**
**Königsteiner Strasse 129**
**W-6230 Frankfurt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft chromophore Peptide der allgemeinen Formel I

DIS-D-Ala-Pro-R (I)

worin R für Gly-OH oder $NH_2$ steht und
DIS 5-Dimethylamino-naphthalin-1-sulfonyl bedeutet, sowie deren Salze.

Als Salze kommen insbesondere Alkali- und Erdalkalisalze, Salze mit Aminen und Salze mit anorganischen oder organischen Säuren wie beispielsweise Salzsäure, Bromwasserstoffsäure, Schwefelsäure, Maleinsäure oder Fumarsäure in Betracht.

Die peptidylglycin-α-amidierende Monooxygenase (PAM) ist ein wichtiges Enzym, das aus Peptiden mit C-terminalem Glycin (Peptidylglycin) die entsprechenden Peptidamide bildet. Bei vielen Peptidhormonen, wie z.B. beim Gonadoliberin, Thyreoliberin, Secretin oder Calcitonin ist die amidierte Form aktiver. Allerdings werden nur Peptide mit neutralen C-terminalen Aminosäuren amidiert (A.F. Bradbury, M.D.A. Finnie und D.G. Smyth, Nature 298 (1982) 686-688).

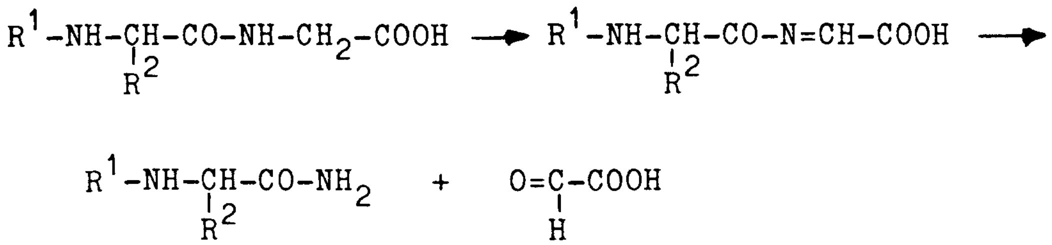

So fand man, daß dieses Enzym z.B. für die Bildung des Thyreotropin-Releasing Faktor (TRH) (I. Husain und S.S. Tate, FEBS Letters 152 (1983) 277-281; J.S. Kizer, W.H. Busby, Jr., C. Cottle and W.W. Youngblood, Proc. Natl. Acad. Sci. USA 81 (1984) 3228-3232; S. Gomez, C. di Bello, L.T. Hung, R. Genet, J.-L. Morgat, P. Formageot und P. Cohen, FEBS Letters 167 (1984) 160) und des α-Melanotropins (α-MSH) (C.C. Glembotski, J. Biol. Chem. 259 (1984) 13041-13048) verantwortlich ist.

Diese Monooxygenase ist abhängig von Ascorbinsäure, Kupferionen und Sauerstoff und wird dort gebildet, wo auch Peptidamide ausgeschieden werden. Bisher wurde die peptidylglycin-α-amidierende Monooxygenase vor allem in der Hypophyse und im zentralen Nervensystem gefunden. Aber auch im Plasma läßt sich dieses Enzym nachweisen. Bei peptidamid-bildenden Tumoren ist die Aktivität des Enzyms im Plasma stark erhöht und kann somit als Marker für bestimmte endocrine Tumoren dienen.

Die Aktivität dieses Enzyms kann durch Umwandlung von

$^{125}$J-D-Tyr-Val-Gly-OH → $^{125}$J-D-Tyr-Val-$NH_2$

gemessen werden (G.S. Wand, R.L. Ney, R.E. Mains, B.A. Eipper, Neuroendocrinology 41 (1985) 482-489; B.A. Eipper, A.C. Myers und R.E. Mains, Endocrinology 116 (1985) 2497-2504; G.S. Wand, R.L. Ney, S. Baylin, B. Eipper und R.E. Mains, Metabolism 34 (1985) 1044-1052).

Um nicht radioaktiv arbeiten zu müssen, wurde nach einem neuen Weg zur Bestimmung der peptidylglycin-α-amidierenden Aktivität gesucht.

Diese Aufgabe wurde gelöst durch ein fluoreszierendes Substrat, das mittels chromatographischer Methoden (DC, HPLC) abgetrennt und identifiziert werden kann. Das erfindungsgemäße DIS-D-Ala-Pro-Gly-OH hat neben dem Vorteil seiner fluoreszierenden 5-Dimethylamino-naphthalin-1-sulfonyl-(DIS-) Gruppe auch noch den Vorzug, daß durch den Einbau des Prolins ein C-terminaler Abbau durch Carboxypeptidasen erschwert ist. Das DIS-D-Ala-Pro-$NH_2$ dient als Vergleich.

Die Erfindung betrifft weiterhin ein Verfahren zur Herstellung eines Peptids der Formel I, das dadurch gekennzeichnet ist, daß man eine Verbindung der Formel II

DIS-Hal (II)

in welcher
DIS 5-Dimethylamino-naphthalin-1-sulfonyl und

Hal Halogen, vorzugsweise Chlor, bedeuten,
umsetzt mit einer Verbindung der Formel III

H-D-Ala-Pro-$R^1$ (III)

in welcher
$R^1$ Gly-R oder $NH_2$ und
$R^2$ eine Carboxylschutzgruppe bedeuten,
im Falle, daß $R^1$ Gly-$R^2$ bedeutet, die Carboxylschutzgruppe von den so erhaltenen Verbindungen der Formel IV-unter Bildung der freien Carboxylfunktion abspaltet, und das so erhaltene Peptid der Formel I gegebenenfalls in seine Salze überführt.

Geeignete Carboxylschutzgruppen $R^2$ sind beispielsweise in Schröder, Lübke, The Peptides, Volume I, New York 1965, Seiten 72-75 oder T.W. Greene, "Protective Groups in Organic Synthesis", Wiley-Interscience, New York 1981 beschrieben. Infrage kommen Niederalkylgruppen, wie Methyl, Ethyl oder tert.-Butyl und Aralkylgruppen, wie Benzyl oder p-Nitrobenzyl; bevorzugt ist tert.-Butyl. Die Abspaltung der Schutzgruppe aus den Zwischenprodukten der Formel IV

D IS-D-Ala-Pro-$R^1$ (IV)

in welcher $R^1$ für Gly-$R^2$ steht und DIS und $R^2$ wie oben definiert sind, erfolgt je nach Art der Schutzgruppe durch alkalische oder saure Hydrolyse oder durch Hydrogenolyse. Das erfindungsgemäße Substrat DIS-D-Ala-Pro-Gly-OH wird vorzugsweise aus dem entsprechenden tert.-Butylester durch eine saure Behandlung gewonnen. Die Peptide der oben genannten Formel III können nach den allgemeinen Methoden der Peptidchemie, beispielsweise durch Kupplung entsprechender gegebenenfalls geschützter Fragmente in Gegenwart von Dicyclohexylcarbodiimid und gegebenenfalls 1-Hydroxy-1H-benzotriazol in einem geeigneten Lösungsmittel (vgl. Chem. Ber. 103 [1970] 788, 2024) hergestellt werden.

Die Erfindung betrifft des weiteren ein Verfahren zum qualitativen oder quantitativen Nachweis der peptidylglycin-α-amidierenden Monooxygenase, das dadurch gekennzeichnet ist, daß man eine Lösung der Formel I, worin R Gly-OH bedeutet mit der die peptidylglycin-α-amidierenden Monooxygenase enthaltenden Analysenprobe inkubiert, die entstandene Verbindung der Formel I, worin R $NH_2$ bedeutet, abtrennt und deren Menge quantitativ bestimmt, die Verwendung einer Verbindung der Formel I zum qualitativen oder quantitativen Nachweis der peptidylglycin-α-amidierenden Monooxygenasen, Mittel enthaltend eine Verbindung der Formel I sowie Zwischenprodukte der Formel IV,
in welcher
DIS 5-Dimethylamino-naphthalin-1-sulfonyl,
$R^1$ Gly-$R^2$ und
$R^2$ eine Carboxylschutzgruppe bedeuten.

Das Enzym (PAM) wurde nach einer Methode von Eipper et al. (Peptides 4 (1983) 921-928) aus Rinderhypophysen isoliert. Dabei wurden frische Hypophysen in Trispuffer homogenisiert, das Homogenat über eine Percoll-Gradientenzentrifugation aufgetrennt und die Vesikelfraktion isoliert. In dieser Fraktion konnte die höchste Aktivität nachgewiesen werden. Durch Ammonsulfat-Fällung und konsekutive Gelfiltration (Glembotski, Arch. Biochem. Biophys. 241 (1985) 673-683) konnte die Aktivität weiter angereichert werden. Die Versuche mit dem erfindungsgemäßen Substrat der Formel I (R = Gly-OH) und der Vergleichssubstanz der Formel I (R = $NH_2$) wurden sowohl mit den Rohfraktionen als auch mit den angereicherten Fraktionen durchgeführt.

Die nachstehenden Beispiele dienen zur Erläuterung der Erfindung, ohne daß diese darauf beschränkt wäre.

Beispiel 1

DIS-D-Ala-Pro-Gly-OH

a) Z-D-Ala-Pro-Gly-OBut

Zu einer Lösung von 11,4 g Z-D-Ala-OH, 13,5 g H-Pro-Gly-OBut•HCl und 6,9 g HOBt in 100 ml Dimethylformamid gibt man bei 0°C 6,54 ml N-Ethylmorpholin und 10,7 g DCC. Man rührt 1 Stunde bei 0°C, 4 Stunden bei Raumtemperatur und läßt über Nacht bei Raumtemperatur stehen. Der Niederschlag wird abgesaugt und das Filtrat eingeengt. Der Rückstand wird zwischen Essigester und Wasser verteilt. Die

Essigesterphase wird nacheinander mit gesättigter wäßriger $NaHCO_3$-Lösung, $KHSO_4/K_2SO_4$-Puffer und Wasser ausgeschüttelt, über $Na_2SO_4$ getrocknet und eingeengt. Die Substanz wird auf 300 g Kieselgel chromatographiert (1. $CH_2Cl_2$/Aceton im Verhältnis 9:1; 2. $CH_2Cl_2/CH_3OH$ im Verhältnis 9,6:0,4). Ausbeute an reiner amorpher Substanz: 10,3 g.

b) H-D-Ala-Pro-Gly-OBut•HCl

10 g Z-D-Ala-Pro-Gly-OBut werden in 100 ml Methanol gelöst und mit Pd-Katalysator unter pH-Kontrolle (Autotitrator mit methanolischer HCl bei pH 4,5) hydriert. Nachdem alles hydriert ist (DC-Kontrolle in $CH_2Cl_2/CH_3OH$ im Verhältnis 9:1) wird der Katalysator abgesaugt und das Filtrat eingeengt. Es bleiben 6,2 g einer farblosen amorphen Substanz zurück.

c) DIS-D-Ala-Pro-Gly-OBut

Zu einer Suspension von 1,68 g (5 mmol) H-D-Ala-Pro-Gly-OBut•HCl und 1,4 g (5,2 mmol) DIS-Chlorid in 25 ml abs. Tetrahydrofuran gibt man bei -5° C 1,4 ml (10 mmol) Triethylamin. Man läßt 1 Stunde bei 0° C und 4 Stunden bei Raumtemperatur rühren und läßt über Nacht bei Raumtemperatur stehen. Der Niederschlag wird abgesaugt und das Filtrat eingeengt. Der Rückstand wird in Essigester gelöst. Die Lösung wird nacheinander mit gesättigter wäßriger $NaHCO_3$-Lösung und Wasser ausgeschüttelt, über $Na_2SO_4$ getrocknet und eingeengt.
Ausbeute: 3,5 g amorphe Substanz.

d) DIS-D-Ala-Pro-Gly-OH

Eine Lösung von 3,5 g DIS-D-Ala-Pro-Gly-OBut in 10 ml 90 %iger Trifluoressigsäure läßt man 1 Stunde bei Raumtemperatur stehen. Danach engt man die Lösung im Vakuum ein. Es bleiben 3,2 g einer amorphen Substanz zurück. Zur Reinigung werden 300 mg über eine Kieselgelsäule (40 x 4 cm) chromatographiert. Elutionsmittel: $CH_2Cl_2/CH_3OH/CH_3COOH/H_2O$ im Verhältnis 9:1:0,1:0,1. Die Fraktionen mit der reinen Substanz werden eingeengt. Der Rückstand wird in Wasser gelöst und gefriergetrocknet.
Ausbeute: 191 mg; $[\alpha]_D^{24}$ = -51,8° (c = 1, in 5%igerwäßriger Essigsäure).
$^1$H-NMR (270 MHz) in $^2$H-DMSO: Charakteristische Signale bei $\delta$ = 1,0 [d, 3H, $-CH_3$ (Ala)]; 2,83 [2s, 6H, -N-$(CH_3)_2$ (DIS)]; 3,7 [d, 2H, $-CH_2-$ (Gly)]; 7,25-8,5 [d, m, 8H (2 = NH und 6 arom. H von DIS)].

Beispiel 2

DIS-D-Ala-Pro-$NH_2$

a) Z-D-Ala-Pro-$NH_2$

Zu einer Lösung von 11,2 g (50 mmol) Z-D-Ala-OH, 7,53 g (50 mmol) H-Pro-$NH_2$•HCl und 6,8 g (50 mmol) HOBt in 100 ml Dimethylformamid gibt man bei 0° C 6,4 ml (50 mmol) N-Ethylmorpholin und 11,4 g (55 mmol) Dicyclohexylcarbodiimid (DCC). Man läßt 1 Stunde bei 0° C und 5 Stunden bei Raumtemperatur rühren und läßt über Nacht bei Raumtemperatur stehen. Der Niederschlag wird abgesaugt und das Filtrat eingeengt. Der Rückstand wird zwischen Essigester und Wasser verteilt. Die Essigesterphase wird nacheinander mit gesättigter wäßriger $NaHCO_3$-Lösung und $KHSO_4/K_2SO_4$-Puffer und Wasser gewaschen, über $Na_2SO_4$ getrocknet und eingeengt. Der Rückstand wird mit Petrolether mehrmals digeriert und anschließend über 180 g Kieselgel chromatographiert ($CH_2Cl_2/CH_3OH$ im Verhältnis 9,5:0,5). Ausbeute an reinem Peptid: 6,74 g farblose amorphe Substanz.

b) H-D-Ala-Pro-$NH_2$•HCl

6,4 g (20 mmol) Z-D-Ala-Pro-$NH_2$ werden in Methanol gelöst und wie bei 1b katalytisch hydriert. Der Rückstand wird mit Ether verrieben.
Ausbeute: 4,11 g einer farblosen Substanz.

c) DIS-D-Ala-Pro-$NH_2$

Zu einer Suspension von 0,442 g (2 mmol) H-D-Ala-Pro-$NH_2$ und 0,6 g (2,2 mmol) DIS-Chlorid in 15 ml

abs. Tetrahydrofuran gibt man bei 0° C 0,56 ml (4 mmol) Triethylamin. Man läßt 1 Stunde bei 0° C und 5 Stunden bei Raumtemperatur rühren und läßt über Nacht bei Raumtemperatur stehen. Aufarbeitung wie bei 1c. Reinigung an Kieselgel (40 x 4 cm; Elutionsmittel $CH_2Cl_2/CH_3OH$ im Verhältnis 9,5:0,5).
Ausbeuten an reiner Substanz: 0,23 g $[\alpha]_D^{24}$ = -18,4° (c=1, in 5%iger wäßriger Essigsäure).
$^1$H-NMR (270 MHz) in $^2$H-DMSO: Charakteristische Signale bei $\delta$ = 0,97 [d, 3H, $-CH_3$ (Ala)]; 2,83 [2s, 6H, $-N(CH_3)_2$ (DIS)]; 6,9-8,5 [d, m, 9H, 3 = NH und 6 arom. H von DIS].

Beispiel 3

Durchführung des Enzym-Assays mit dem Substrat der Formel I (R = Gly-OH) und der Referenz-Substanz der Formel II (R = $NH_2$)

Die Enzymtests werden in einem Puffer von pH 7,4 durchgeführt. Der wäßrige Puffer besteht aus der Puffersubstanz N-[Tris-(hydroxymethyl)-methyl]-2-amino-ethansulfonsäure (100 mM), $CuSO_4$ (3 $\mu$M), Peptidsubstrat der Formel I (R = Gly-OH) (50 $\mu$M) und Katalase aus Rinderleber (100 $\mu$g/ml). 100 $\mu$l dieser Pufferlösung werden mit 100 $\mu$l der PAM-haltigen Lösung inkubiert und nach 3 Stunden bei 37° C durch Zugabe von 700 $\mu$l $CH_2Cl_2$ gestoppt. Durch kurzes Ausschütteln wird das Peptidamid selektiv in die organische Phase überführt (Extraktionsausbeute: ca. 60 %). Nach Zentrifugation und Abnahme der organischen Phase wird diese in einer Vakuumzentrifuge eingedampft und in einem konstanten Volumen $CH_2Cl_2$ wieder aufgenommen. Zur Vermeidung von Photo-Reaktionen des lichtempfindlichen Substrats werden alle Operationen unter Lichtausschluß durchgeführt. Die erhaltenen Lösungen werden mit einem Autospotter auf HPTLC-Platten (Merck Si 60) aufgetragen und mit Hilfe eines Laufmittelgemisches ($CH_2Cl_2/CH_3OH/H_2O/CH_3COOH$ im Verhältnis 90:10:1:1) getrennt. Die Quantifizierung des entstandenen Amids der Formel I (R = $NH_2$) mit einem Dünnschichtscanner wird ermöglicht durch gleichzeitiges Auftragen des Peptidamids der Formel II in unterschiedlicher Konzentration als Referenz.
Zeitabhängigkeit des Umsatzes von DIS-D-Ala-Pro-Gly-OH mit PAM-Aktivität:

| t (min.): | 60 | 120 | 180 |
|---|---|---|---|
| Peptidamidbildung (ng): | 21,5 | 43,8 | 67,5 |

Nach 3 Stunden sind ca. 5 % des Substrates der Formel I (R = Gly-OH) umgesetzt bei einem Umsatz von 0,29 ng/Stunde pro 1 $\mu$g zugesetztes Protein mit PAM-Aktivität.

## Patentansprüche
**Patentansprüche für folgende Vertragsstaaten : BE, CH, DE, FR, GB, IT, LI, LU, NL, SE**

**1.** Peptid der Formel I

DIS-D-Ala-Pro-R (I)

in welcher
R Gly-OH oder $NH_2$ und
DIS 5-Dimethylamino-naphthalin-1-sulfonyl bedeuten,
sowie deren Salze.

**2.** Verfahren zur Herstellung eines Peptids gemäß Anspruch 1, dadurch gekennzeichnet, daß man eine Verbindung der Formel II

DIS-Hal (II)

in welcher
DIS 5-Dimethylamino-naphthalin-1-sulfonyl und
Hal Halogen bedeuten, umsetzt mit einer Verbindung der Formel III

H-D-Ala-Pro-$R^1$ (III)

in welcher

$R^1$ Gly-$R^2$ oder $NH_2$ und
$R^2$ eine Carboxylschutzgruppe bedeuten,
im Falle, daß $R^1$ Gly-$R^2$ bedeutet, die Carboxylschutzgruppe unter Bildung der freien Carboxylfunktionen abspaltet und das so erhaltene Peptid der Formel I gegebenenfalls in sein Salz überführt.

3. Verfahren zum qualitativen oder quantitativen Nachweis der peptidylglycin-α-amidierenden Monooxygenase, dadurch gekennzeichnet, daß man eine Lösung einer Verbindung gemäß Anspruch 1, worin R Gly-OH bedeutet mit der die peptidylglycin-α-amidierenden Monooxygenase enthaltenden Analysenprobe inkubiert, die entstandene Verbindung gemäß Anspruch 1, worin R $NH_2$ bedeutet abtrennt und deren Menge quantitativ bestimmt.

4. Verwendung einer Verbindung gemäß Anspruch 1 zum qualitativen oder quantitativen Nachweis der peptidylglycin-α-amidierenden Monooxygenasen.

5. Mittel zum qualitativen oder quantitativen Nachweis der peptidylglycin-α-amidierenden Monooxygenase, gekennzeichnet durch einen ausreichenden Gehalt an einer Verbindung der Formel I von Anspruch 1.

6. Peptid der Formel IV

DIS-D-Ala-Pro-$R^1$ (IV)

in welcher
DIS 5-Dimethylamino-naphthalin-1-sulfonyl
$R^1$ Gly-$R^2$ und
$R^2$ eine Carboxylschutzgruppe bedeuten.

**Patentansprüche für folgende Vertragsstaaten : AT, ES**

1. Verfahren zur Herstellung eines Peptids der Formel I

DIS-D-Ala-Pro-R (I)

in welcher
R Gly-OH oder $NH_2$ und
DIS 5-Dimethylamino-naphthalin-1-sulfonyl bedeuten,
sowie deren Salze,
dadurch gekennzeichnet, daß man eine Verbindung der Formel II

DIS-Hal (II)

in welcher
DIS 5-Dimethylamino-naphthalin-1-sulfonyl und
Hal Halogen bedeuten, umsetzt mit einer Verbindung der Formel III

H-D-Ala-Pro-$R^1$ (III)

in welcher
$R^1$ Gly-$R^2$ oder $NH_2$ und
$R^2$ eine Carboxylschutzgruppe bedeuten,
im Falle, daß $R^1$ Gly-$R^2$ bedeutet, die Carboxylschutzgruppe unter Bildung der freien Carboxylfunktionen abspaltet und das so erhaltene Peptid der Formel I gegebenenfalls in sein Salz überführt.

2. Verfahren zum qualitativen oder quantitativen Nachweis der peptidylglycin-α-amidierenden Monooxygenase, dadurch gekennzeichnet, daß man eine Lösung einer Verbindung gemäß Anspruch 1, worin R Gly-OH bedeutet mit der die peptidylglycin-α-amidierenden Monooxygenase enthaltenden Analysenprobe inkubiert, die entstandene Verbindung gemäß Anspruch 1, worin R $NH_2$ bedeutet abtrennt und deren Menge quantitativ bestimmt.

**3.** Verfahren zur Herstellung eines Peptids der Formel IV

DIS-D-Ala-Pro-$R^1$ (IV)

in welcher
DIS 5-Dimethylamino-naphthalin-1-sulfonyl
$R^1$ Gly-$R^2$ und
$R^2$ eine Carboxylschutzgruppe bedeuten,
dadurch gekennzeichnet, daß man eine Verbindung der Formel II

DIS-Hal (II)

in welcher
DIS 5-Dimethylamino-naphthalin-1-sulfonyl und
Hal Halogen bedeuten, umsetzt mit einer Verbindung der Formel III

H-D-Ala-Pro-$R^1$ (III)

in welcher
$R^1$ Gly-$R^2$ und
$R^2$ eine Carboxylschutzgruppe bedeuten.

**4.** Mittel zum qualitativen oder quantitativen Nachweis der peptidylglycin-α-amidierenden Monooxygenase, gekennzeichnet durch einen ausreichenden Gehalt an einer Verbindung der Formel I von Anspruch 1.

**Claims**
**Claims for the following Contracting States : BE, CH, DE, FR, GB, I, LI, LU, NL, SE**

**1.** A peptide of the formula I

DIS-D-Ala-Pro-R (I)

in which
R denotes Gly-OH or $NH_2$ and
DIS denotes 5-dimethylaminonaphthalene-1-sulfonyl,
and salts thereof.

**2.** A process for the preparation of a peptide as claimed in claim 1, which comprises reacting a compound of the formula II

DIS-Hal (II)

in which
DIS denotes 5-dimethylaminonaphthalene-1-sulfonyl
and
Hal denotes halogen, with a compound of the formula III

H-D-Ala-Pro-$R^1$ (III)

in which
$R^1$ denotes Gly-$R^2$ or $NH_2$ and
$R^2$ denotes a carboxyl protective group,
in the event that $R^1$ denotes Gly-$R^2$, splitting off the carboxyl protective group with the formation of the free carboxyl groups, and, if appropriate, converting the peptide of the formula I thus obtained into its salt.

**3.** A process for the qualitative or quantitative detection of peptidylglycine-α-amidizing monooxygenase, which comprises incubating a solution of a compound as claimed in claim 1, in which R denotes Gly-OH with the analytical sample containing the peptidylglycine-α-amidizing monooxygenase, separating off the resulting compound as claimed in claim 1, in which R denotes $NH_2$, and determining the amount thereof quantitatively.

**4.** The use of a compound as claimed in claim 1, for the qualitative or quantitative detection of peptidylglycine-α-amidizing monooxygenases.

**5.** An agent for the qualitative or quantitative detection of peptidylglycine-α-amidizing monooxygenase, which has an adequate content of a compound of the formula I as claimed in claim 1.

**6.** A peptide of the formula IV

DIS-D-Ala-Pro-$R^1$ (IV)

in which
DIS denotes 5-dimethylaminonaphthalene-1-sulfonyl,
$R^1$ denotes Gly-$R^2$ and
$R^2$ denotes a carboxyl protective group.

**Claims for the following Contacting States : AT, ES**

**1.** A process for the preparation of a peptide of the formula I

DIS-D-Ala-Pro-R (I)

in which
R denotes Gly-OH or $NH_2$ and
DIS denotes 5-dimethylaminonaphthalene-1-sulfonyl,
and salts thereof, which comprises reacting a compound of the formula II

DIS-Hal (II)

in which
DIS denotes 5-dimethylaminonaphthalene-1-sulfonyl
and
Hal denotes halogen, with a compound of the formula III

H-D-Ala-Pro-$R^1$ (III)

in which
$R^1$ denotes Gly-$R^2$ or $NH_2$ and
$R^2$ denotes a carboxyl protective group,
in the event that $R^1$ denotes Gly-$R^2$, splitting off the carboxyl protective group with the formation of the free carboxyl groups, and, if appropriate, converting the peptide of the formula I thus obtained into its salt.

**2.** A process for the qualitative or quantitative detection of peptidylglycine-α-amidizing monooxygenase, which comprises incubating a solution of a compound as claimed in claim 1, in which R denotes Gly-OH with the analytical sample containing the peptidylglycine-α-amidizing monooxygenase, separating off the resulting compound as claimed in claim 1, in which R denotes $NH_2$, and determining the amount thereof quantitatively.

**3.** A process for the preparation of a peptide of the formula IV

DIS-D-Ala-Pro-$R^1$ (IV)

in which
DIS denotes 5-dimethylaminonaphthalene-1-sulfonyl,

$R^1$ denotes Gly-$R^2$ and
$R^2$ denotes a carboxyl protective group,
which comprises reacting a compound of the formula II

DIS-Hal (II)

in which
DIS denotes 5-dimethylaminonaphthalene-1-sufonyl and
Hal denotes halogen, with a compound of the formula III

H-D-Ala-Pro-$R^1$ (III)

in which
$R^1$ denotes Gly-$R^2$ and
$R^2$ denotes a carboxyl protective group.

**4.** An agent for the qualitative or quantitative detection of peptidylglycine-$\alpha$-amidizing monooxygenase, which has an adequate content of a compound of the formula I as claimed in claim 1.

**Revendications**
**Revendications pour les Etats contractants suivants : BE, CH, DE, FR, GB, IT, LI, LU, NL, SE**

**1.** Peptide de formule I

DIS-D-Ala-Pro-R (I),

dans laquelle
R représente Gly-OH ou $NH_2$, et
DIS est le groupe 5-diméthylamino-naphtalène-1-sulfonyle, ainsi que ses sels.

**2.** Procédé de fabrication d'un peptide selon la revendication 1, caractérisé en ce qu'on fait réagir un composé de formule II

DIS-Hal (II),

dans laquelle
DIS est le groupe 5-diméthylamino-naphtalène-1-sulfonyle, et
Hal représente un atome d'halogène,
avec un composé de formule III

H-D-Ala-Pro-$R^1$ (III),

dans laquelle
$R^1$ représente Gly-$R^2$ ou $NH_2$, et
$R^2$ est un groupe protecteur du carboxyle,
et que, dans le cas où $R^1$ représente Gly-$R^2$, on clive le groupe protecteur du carboxyle, avec formation de la fonction carboxyle libre, et qu'on transforme éventuellement en son sel le peptide de formule I ainsi obtenu.

**3.** Procédé d'identification qualitative ou quantitative de la monooxygénase $\alpha$-amidante de peptidylglycine, caractérisé en ce qu'on fait incuber une solution d'un composé selon la revendication 1, dans lequel R est Gly-OH, avec l'échantillon à analyser contenant la monooxygénase $\alpha$-amidifiante de peptidylglycine, on sépare le composé selon la revendication 1 formé, dans lequel R est $NH_2$, et on le dose.

**4.** Utilisation d'un composé selon la revendication 1 pour l'identification qualitative ou quantitative de monooxygénases $\alpha$-amidifiantes de peptidylglycines.

**5.** Réactif pour l'identification qualitative ou quantitative de la monooxygénase $\alpha$-amidifiante de peptidyl-

glycine, caractérisé par une teneur suffisante en un composé de formule I de la revendication 1.

**6.** Peptide de formule IV

DIS-D-Ala-Pro-$R^1$ (IV),

dans laquelle
DIS est le groupe 5-diméthylamino-naphtalène-1-sulfonyle,
$R^1$ représente Gly-$R^2$, et
$R^2$ est un groupe protecteur du carboxyle.

**Revendications pour les Etats contractants suivants : AT, ES**

**1.** Procédé de fabrication d'un peptide de formule I

DIS-D-Ala-Pro-R (I),

dans laquelle
R représente Gly-OH ou $NH_2$, et
DIS est le groupe 5-diméthylamino-naphtalène-1-sulfonyle, ainsi que ses sels,
caractérisé en ce qu'on fait réagir un composé de formule II

DIS-Hal (II),

dans laquelle
DIS est le groupe 5-diméthylamino-naphtalène-1-sulfonyle, et
Hal représente un atome d'halogène,
avec un composé de formule III

H-D-Ala-Pro-$R^1$ (III),

dans laquelle
$R^1$ représente Gly-$R^2$ ou $NH_2$, et
$R^2$ est un groupe protecteur du carboxyle,
et que, dans le cas où $R^1$ représente Gly-$R^2$, on clive le groupe protecteur du carboxyle, avec formation de la fonction carboxyle libre, et qu'on transforme éventuellement en son sel le peptide de formule I ainsi obtenu.

**2.** Procédé d'identification qualitative ou quantitative de la monooxygénase $\alpha$-amidante de peptidylglycine, caractérisé en ce qu'on fait incuber une solution d'un composé selon la revendication 1, dans lequel R est Gly-OH, avec l'échantillon à analyser contenant la monooxygénase $\alpha$-amidifiante de peptidylglycine, on sépare le composé selon la revendication 1 formé, dans lequel R est $NH_2$, et on le dose.

**3.** Procédé de préparation d'un peptide de formule IV

DIS-D-Ala-Pro-$R^1$ (IV),

dans laquelle
DIS est le groupe 5-diméthylamino-naphtalène-1-sulfonyle,
$R^1$ représente Gly-$R^2$, et
$R^2$ est un groupe protecteur du carboxyle,
caractérisé en ce qu'on fait réagir un composé de formule II

DIS-Hal (II),

dans laquelle
DIS est le groupe 5-diméthylamino-naphtalène-1-sulfonyle, et
Hal représente un atome d'halogène,

avec un composé de formule III

H-D-Ala-Pro-$R^1$ (III),

dans laquelle
$R^1$ représente Gly-$R^2$, et
$R^2$ est un groupe protecteur du carboxyle.

**4.** Réactif pour l'identification qualitative ou quantitative de la monooxygénase $\alpha$-amidifiante de peptidyl-glycine, caractérisé par une teneur suffisante en un composé de formule I de la revendication 1.